Europäisches Patentamt

European Patent Office

Office européen des brevets

(11) Veröffentlichungsnummer: **0 339 101 B1**

# EUROPÄISCHE PATENTSCHRIFT

(45) Veröffentlichungstag der Patentschrift: **16.10.91**

(21) Anmeldenummer: **88106630.2**

(22) Anmeldetag: **26.04.88**

(51) Int. Cl.5: **A61K 31/70**, //(A61K31/70, 31:55)

(54) Mittel zur Beeinflussung des Blutzuckerspiegels bei Diabetes.

(43) Veröffentlichungstag der Anmeldung:
02.11.89 Patentblatt 89/44

(45) Bekanntmachung des Hinweises auf die
Patenterteilung:
16.10.91 Patentblatt 91/42

(84) Benannte Vertragsstaaten:
AT BE CH ES FR GB IT LI NL SE

(56) Entgegenhaltungen:
DE-A- 3 711 723

UNLISTED DRUGS, Band 36, Nr. 3, März 1984,
Seite 49, Chatham, New Jersey, US

UNLISTED DRUGS, Band 36, Nr. 7, Juli 1984,
Seite 131, Chatham, New Jersey, US

CHEMICAL ABSTRACTS, Band 85, Nr. 23, 6.
Dezember 1976, Seite 311, Zusammenfassung Nr. 174738s, Columbus, Ohio, US; L.
SACCA et al.: "Some peculiarities of the
glucoregulatory response to glucose infusion in the rat", & ACTA DIABETOL. LAT.
1976, 13(1-2), 1-7

(73) Patentinhaber: Bergis, Kristian, Dr.
Theodor-Klotzbücher-Strasse 10
W-6990 Bad Mergentheim(DE)

Patentinhaber: Boerner, Harro
Kleinfeldstrasse 41 A
W-8100 Garmisch-Partenkirchen(DE)

Patentinhaber: Quadbeck, Jürgen
von Berlichingen-Strasse 17
W-6990 Bad Mergentheim(DE)

(72) Erfinder: Bergis, Kristian, Dr.
Theodor-Klotzbücher-Strasse 10
W-6990 Bad Mergentheim(DE)
Erfinder: Boerner, Harro
Kleinfeldstrasse 41 A
W-8100 Garmisch-Partenkirchen(DE)
Erfinder: Quadbeck, Jürgen
von Berlichingen-Strasse 17
W-6990 Bad Mergentheim(DE)

(74) Vertreter: Patentanwälte Phys. Bartels
Dipl.-Ing. Fink Dr.-Ing. Held
Lange Strasse 51
W-7000 Stuttgart 1(DE)

**Beschreibung**

Die Erfindung betrifft ein Mittel zur Beeinflussung des Blutzuckerspiegels bei Diabetes, insbesondere bei insulinpflichtigem Diabetes, unter Verwendung von Glukose.

Beim insulinpflichtigen Diabetes stellt die Hypoglykämie ein ständiges Problem dar, da sie selbst bei einer guten Einstellung des Insulindiabetikers hinsichtlich ihrer Häufigkeit und ihres Schweregrades prinzipiell nie vorhersehbar ist und stets eine Soforttherapie verlangt mit dem Ziel der Normalisierung des Blutzuckerspiegels und der Vermeidung der nachfolgenden endogenen hypoglykämischen Gegenregulationen. Je nach Einstellgüte und aktueller Blutzuckerlage verfügt nämlich auch der diabetische Organismus noch über ein Warnsystem für den Blutzuckerrückgang. Bei Werten zwischen 70 und 60 mg/dl werden automatisch hormonelle Gegenmaßnahmen getroffen, die den Blutzuckerspiegel mit körpereignen Mitteln wieder ansteigen lassen. Es kommt dabei zur hormonell und nervlich gesteuerten Freisetzung von Hormonen, die vor allem das Leberglykogen mobilisieren und kurzfristig blutzuckersteigernd wirksam sing. Unter diesen Hormonen spielt neben dem Adrenalin, dem Cortisol und dem Glukagon das Wachstumshormon (STH) eine wichtige Rolle, insbesondere im Kindes- und Judendalter. Es wird über cholinerge Fasern des zentralen Nervensystems aktiviert und bei Unterzuckerung, aber auch bei Insulinmangel, verstärkt freigesetzt. Man ist deshalb bestrebt, eine Hypoglykämie durch eine Glukosegabe schon im Anfangsstadium zu stoppen und damit nachfolgende endogene hypoglykämische Gegenreaktionen zu vermeiden. Allerdings ist dies bisher in vielen Fällen nicht gelungen, weil Diabetiker oft nicht früh genug die beginnende Hypoglykämie erkennen oder bekämpfen.

Der Erfindung liegt deshalb die Aufgabe zugrunde, ein Mittel zu schaffen, das es ermöglicht, einer stabilen Einstellung vieler Insulin-Diabetiker wesentlich näher zu kommen, als dies bisher möglich war.

Diese Aufgabe löst ein Mittel gemäß Anspruch 1.

Durch die Beigabe eines Anticholinergikums zu Glukose, die wegen der raschen Resorption durch den Organismus ein schnell wirkendes Mittel gegen eine Hypoglykämie darstellt, läßt sich mit dem erfindungsgemäßen Mittel nicht nur rasch der abgesunkene Blutzuckerspiegel wieder erhöhen. Vor allem wird die unterzuckerungsbedingte Gegenregulation zu einem kontrollierbaren Vorgang mit dem Ergebnis einer behutsamen Blutzuckerpluskorrektur ohne die Gefahr eines überschießenden Blutzuckeranstieges, wie er häufig bei ausgeprägten Gegenregulationen beobachet wird.

Diese Gegenregulationen sind immer Ausdruck einer vermehrten Aktivität der kontrainsulinären Hormone, wobei die unterschiedlich langen Wirkzeiten dieser Stoffe eine wichtige Rolle spielen, Dem Wachstumshormon (STH) kommen hier besondere, offensichtlich auch prolongierende Funktionen zu. Ein typischer Fall für das aktuelle Überwiegen von Wachstumshormonen im Blutzuckereinstellmechanismus ist das sogenannte Dawn-Phänomen, bei welchem in den frühen Morgenstunden Blutzuckeranstiege weit über das übliche Maß hinaus beobachtet werden aufgrund von Insulinmangel und bei vermehrter Ausschüttung des Wachstumshormons. Diese scheint bezüglich eines insulinantagonistischen Effektes besonders stark wirksam zu sein und begünstigt Blutzuckeranstiege zur genannten Zeit. Durch die erfindungsgemäße Beigabe eines Anticholinergikums zu Glukose läßt sich ein Anstieg des Wachstumshormon-Spiegels in Folge einer Absenkung des Blutzuckerspiegels in bemerkenswertem Ausmaße unterdrücken. Dies ist darauf zurückzuführen, daß zentrale Neurone vorwiegend durch Muscarin-Rezeptoren erregt werden. Die Ausschüttung von STH aus dem Hypothalamus bzw. der Hypophyse scheint durch zentralnervöse Neurone und Muscarin-Rezeptoren gesteuert zu sein. Muscarin-Rezeptor-Antagonisten, also anticholinerge Wirkstoffe, sind demzufolge in der Lage, zentralnervöse Impulse, die zum Ausstoß von STH führen, zu behindern oder sogar zu blokkieren.

Ein bevorzugtes Anticholinergikum ist Pirenzepin.

Um bequem verabreicht und rasch resorbiert werden zu können, sieht man zweckmäßigerweise die Glukose in flüssiger Form vor. Sie kann zusammen mit dem Anticholinergikum oral verabreicht werden.

Das erfindungsgemäße Mittel ist vorzugsweise in einer Verpakkungseinheit konfektioniert, die sowohl die Glukose, beispielsweise 20g in 15 bis 20ml Wasser, als auch das Anticholinergikum in einer dosisgerechten Menge von vorzugsweise 50 mg enthält, um die als auch das Anticholinergikum in dosisgerechter Menge enthält, um die Hypoglykämie rasch zu beseitigen und eine Gegenregulation zu unterbinden. Dadurch können durch das erfindungsgemäße Mittel nicht nur starke Blutzuckerspiegelabsenkungen, sondern auch starke Blutzuckerspiegelanhebungen, sicher vermieden werden.

Bei Vergleichsversuchen wurden einer ersten Gruppe von insulinpfichtigen Type-I-Diabetikern zur Beseitigung einer aufgetretenen Hypoglykämie 60 ml Glukose mit einem Gehalt von 20 g gelöstem Traubenzucker oral verabreicht. Der STH-Spiegel lag zum Zeitpunkt der Verabreichung im Mittel bei 3,49 ng/ml. Eine Stunde nach der Verabreichung lag der STH-Spiegel im Durchschnitt bei 2,68 ng/ml. Einer zweiten Gruppe wurde zusammen mit den 60 ml Glukose 20 mg Pirenzipin-Dihydrochlorid, gelöst in Propylenglykol, verabreicht. Auch bei dieser Gruppe betrug bei der Verabreichung der Mittelwert des STH-Spiegels 3,49 ng/ml. Eine Stunde nach der Verabreichung betrug der Mittelwert des STH-Spiegels jedoch nur noch 0,93 ng/ml.

Durch eine Konfektionierung des erfindungsgemäßen Mittels in einer Verpackungseinheit eignet es sich auch vorzüglich zur Selbsttherapie.

Für Personen, bei denen Hypoglykämie zu einem Funktionsmangel bestimmter Hirnstrukturen führen kann oder führt, kann außer dem Anticholinergikum ein Motilitätsförderer beigegeben sein. Hierdurch kann eine Beeinträchtigung der Motorik vermieden oder rasch wieder beseitigt werden. Dies gilt auch für psychische Fehlreaktionen und die Gefahren für das zentrale Nervensystem, die bei einer Unterzuckerung autreten können.

Als chemische Motilitätsförderer kommen beispielsweise Metoclopramid, Bromoprid, Domperidon oder Alizaprid in Frage. Als phytotherapeutische oder pflanzliche Motilitätsförderer sind Medikamente auf der Basis von Iberis amara, also beispielsweise Iberogast oder Digestodoron, geeignet.

## Patentansprüche

1. Mittel zur Beeinflussung des Blutzuckerspiegels bei Diabetes, insbesondere bei insulinpflichtigem Diabetes, unter Verwendung von Glukose, dadurch gekennzeichnet, daß es ein Anticholinergikum als Beigabe enthält.

2. Mittel nach Anspruch 1, gekennzeichnet durch Pirenzepin als Anticholinergikum.

3. Mittel nach Anspruch 1 oder 2, dadurch gekennzeichnet, daß die Glukose sich in flüssiger Form befindet.

4. Mittel nach einem der Ansprüche 1 bis 3, dadurch gekennzeichnet, daß es zusätzlich einen Motilitätsförderer als Beigabe enthält.

5. Mittel nach einem der Ansprüche 1 bis 4, dadurch gekennzeichnet, daß es in einer Verpackungeinheit konfektioniert ist.

## Claims

1. A means of influencing the blood sugar level in diabetes, particularly in the case of insulin-controlled diabetes, using glucose, characterised in that it contains an anticholinergicum.

2. A means according to Claim 1, characterised by pirenzepin as an anticholinergicum.

3. A means according to Claim 1 or 2, characterised in that the glucose is present in a liquid form.

4. A means according to one of Claims 1 to 3, characterised in that it also contains an added motility promoter.

5. A means according to one of Claims 1 to 4, characterised in that it is made up in a packaging unit.

## Revendications

1. Agent pour influencer le taux sanguin du sucre dans le diabète, en particulier dans le diabète insulino-dépendant, utilisant du glucose, caractérisé en ce qu'il contient comme additif un anticholinergique.

2. Agent selon la revendication 1, caractérisé en ce que l'anticholinergique est de la Pirenzepine.

3. Agent selon l'une des revendications 1 ou 2, caractérisé en ce que le glucose se trouve sous forme liquide.

4. Agent selon l'une quelconque des revendications 1 à 3, caractérisé en ce qu'il contient en outre comme additif un renforçateur de motilité.

5. Agent selon l'une quelconque des revendications 1 à 4, caractérisé en ce qu'il est confectionné en une unité de conditionnement.